# EUROPEAN PATENT SPECIFICATION

(11) **EP 1 604 619 B1**
(45) Date of publication and mention of the grant of the patent: **08.08.2012**
(21) Application number: 05012522.8
(22) Date of filing: 10.06.2005
(51) Int. Cl.: A61B 17/80

(54) **Bone plating system**
Knochenplattensystem
Système de plaque d'ostéosynthèse

(30) Priority: 10.06.2004 US 865248
(43) Date of publication of application: 14.12.2005
(73) Proprietor: EBI, L.P., Parsippany, NJ 07054 (US)
(72) Inventor: Leung, Takkwong Ross, Piscataway New Jersey 08854 (US); Waliluk, Stephen, Phillipsburg New Jersey 08865 (US); Bailey, Kirk, Blairstown New Jersey 07825 (US)
(74) Representative: Duxbury, Stephen

(56) References cited:
- DE-U1- 20 317 651
- US-A1- 2001 014 807
- US-B1- 6 730 091

## Description

### CROSS-REFERENCE TO RELATED APPLICATIONS

### FIELD OF THE INVENTION

The present invention relates generally to orthopedic surgical procedures. More particularly, the present invention relates to a bone plating system. The features of the preamble of claim 1 are known from the document DE-U-203 17 651.

### BACKGROUND AND SUMMARY OF THE INVENTION

In certain orthopedic surgical procedures, it is necessary to secure multiple bones or bone portions relative to each other to facilitate proper healing. For example, it is frequently necessary to secure two or more portions of a broken long bone such as the tibia to ensure proper healing. This need may be the result of physical trauma from fractures or dislocations, degenerative diseases, or tumors. Improper mending of the bone may result in deformity, discomfort or both.

Common methods of fracture treatment include casting and external fixation. It is also well known to treat fractures with internal plating systems. Use of such plating systems involves the attachment of a plate to the bone with bone screws. The plating systems function to stabilize discrete bone portions and thereby facilitate fusion of the bone portions in a particular orientation for healing or to repair a condition of the patient.

Various plating systems are known. One known plating system is shown in German utility model DE 93 21 544 U1. This German utility model illustrates a plating system having a plate member with a shaft portion and a head portion. The head portion is configured to conform to a metaphysis of a bone and includes a plurality of internally threaded holes. The threads of the holes engage threaded heads of bone screws. The shaft portion is shown to include two round holes and an elongated slot. The holes and slot of the shaft portion are unthreaded for receiving bone screws with unthreaded heads.

It remains desirable to continuously improve the pertinent art.

The present invention relates to a plating system for bone as claimed in claim 1.

Additional advantages and further areas of applicability of the present invention will become apparent from the detailed description and appended claims provided hereinafter. It should be understood that the detailed description and specific examples, while indicating the preferred embodiment of the invention, are intended for purposes of illustration only and are not intended to limit the scope of the invention which is defined by the appended claims.

### BRIEF DESCRIPTION OF THE DRAWINGS

The present invention will become more fully understood from the detailed description and the accompanying drawings, wherein:

Figure 1 is a top view of a plating system according to the present invention.

Figure 2 is a cross-sectional view taken along the line 2-2 of Figure 1.

Figure 3 is a cross-sectional view taken along the line 3-3 of Figure 1.

Figure 4 is a cross-sectional view taken along the line 4-4 of Figure 1.

Figure 5 is a cross-sectional view similar to Figure 4, the bone screw shown angulated from the orientation illustrated in Figure 4.

Figure 6 is a cross-sectional view taken along the line 6-6 of Figure 1.

Figure 7 is a cross-sectional view similar to Figure 3A, illustrating a bone screw with an unthreaded head.

Figure 8 is a cross-sectional view similar to Figure 6, illustrating a collar formed with threads to engage the threads of the plate member.

Figure 9 is a cross-sectional view similar to Figure 3, illustrating the first bone screw angulated relative to the plate member.

### DETAILED DESCRIPTION OF EMBODIMENTS

### OF THE PRESENT INVENTION

The following description of embodiments of the present invention will be understood to be merely exemplary in nature and is in no way intended to limit the invention, its application, or uses.

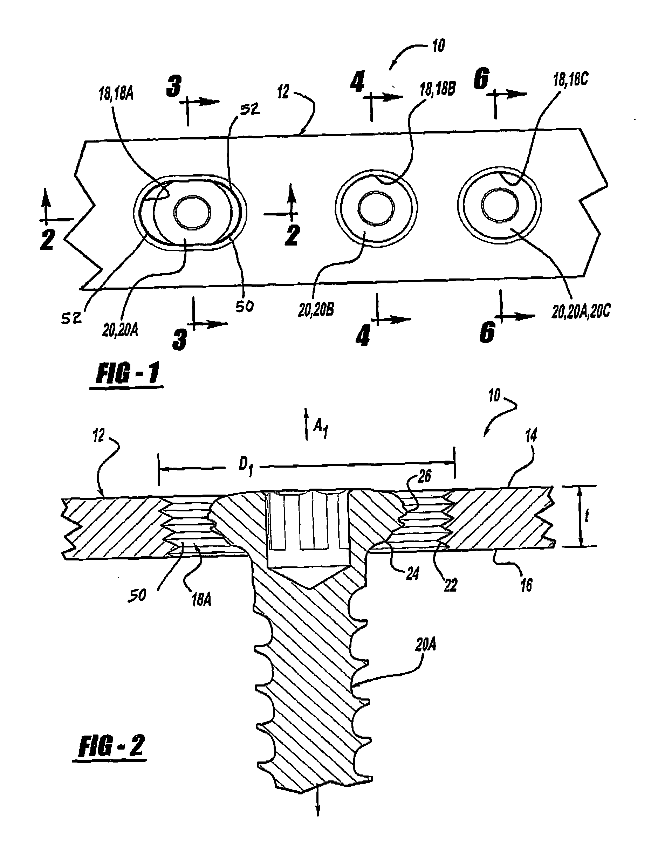

With reference to Figures 1 through 9 of the drawings a bone plating system constructed in accordance with the teachings of the present invention is illustrated and generally identified at reference character 10. In certain applications, the teachings of the present invention are specifically intended for a plating system that can be used to stabilize bone portions of the proximal femur, distal tibia and proximal tibia, for example. It will become apparent to those skilled in the art, however, that the teachings of the present invention are also suitable for various other applications in which surgical repair of bone with a plate is desired. Other applications include but are not limited to the distal radius and the spine.

The bone plating system 10 is illustrated to generally include a plate member 12. The plate member 12 may be flat or may be contoured for specific applications in a manner well known in the art to conform with a bone. The plate member 12 is generally shown to include an upper surface 14 and a lower surface 16. The plate member 12 may be constructed of any suitable biocompatible material. One exemplary material is a titanium alloy such as Ti6Al4V. Other materials having acceptable strength characteristics, including but not limited to stainless steel, may also be employed.

The plate member 12 defines a thickness t between the upper surface 14 and the lower surface 16. The thickness t may be constant throughout the plate member 12 or may be variable. In one particular application in which the plate member 12 is constructed of Ti6Al4V and intended for stabilizing the proximal tibia, the plate member 12 has an approximate thickness between 2.0 and 5.0 mm. More preferably, this particular plate member 12 has a thickness of approximately 2.0 mm. In another particular application, in which the plate member 12 is constructed of stainless steel and intended for stabilizing the distal radius, the plate member 12 has an approximate thickness between 1.5 and 2.0 mm, and more preferably has a thickness of approximately 1.5 mm. It should be readily apparent that the plate thickness t may vary according to material choices and strength requirements.

The plate member 12 is illustrated to define a plurality of holes 18 for receiving bone screws 20 for securing the plate member 12 to a bone (not specifically shown). For purposes of illustration, the plate member 12 is shown in Figure 1 to include three holes 18. It will be understood that the particular number of holes 18 defined by the plate member 12 and the specific types of holes 18 may vary within the scope of the present invention and largely depend on the intended application for the plating system 10.

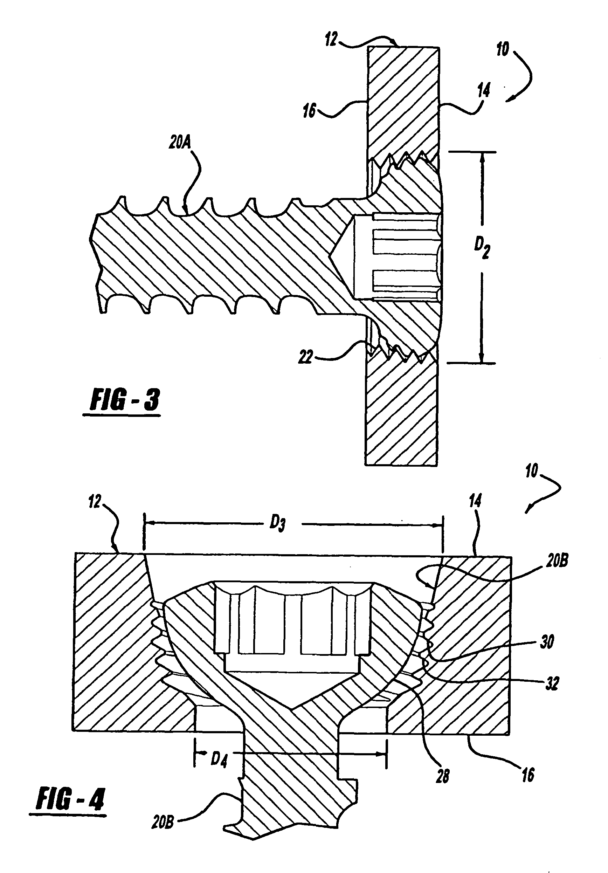

With particular reference to Figures 1-3, the plurality of holes 18 defined by the plate member 12 includes a first hole 18A. The first hole 18A is an elongated hole having circular end portions 52 and is shown in the cross-sectional views of Figures 2 and 3 receiving a first bone screw on fastener 20A of the plurality of bone screws 20. The bone screw 20A has a longitudinal axis A₁ oriented in Figures 2 and 3 generally perpendicular to a plane defined by the plate member 12. The first bone screw 20A is shown to include a threaded shaft for engaging bone. The shaft of the bone screws 20 can be conventional in construction.

The first hole 18A extends between the top surface 14 and the bottom surface 16 in the direction of the axis A₁ (as shown in Figure 2, for example). Given the particular orientation of the bone screw 20A, the axis [[A]] A₁, is coincident with a longitudinal axis of the bone screw 20A. The first hole 18A has a first dimension D₁ adjacent the upper surface 14 in a first direction generally perpendicular to the axis A₁. The first hole 18A includes a second dimension D₂ adjacent the upper surface 14 in a second direction generally perpendicular to the axis A₁ and perpendicular to the first dimension D₁ and to the longitudinal axis of the plate member 12. The first dimension D₁ is substantially greater than the second dimension D₂. In the embodiment illustrated, the first hole 18A is elongated along the length of the plate member 12 and the first and second dimensions D₁ and D₂ correspond to a length and a width of the hole 18A, respectively. In one particular application, the first dimension D₁ is approximately 7.25 mm and the second dimension D₂ is approximately 10.0 mm.

As shown, particularly in Figures 2 and 3, the plate member 12 is threaded. More particularly, the plate member 12 is shown to include at least one ridge 22 on an inner circumferential surface 50 of the first hole 18A, the ridge extending completely about an inner circumference, and thereby forming a closed loop. The plate member 12 may include a plurality of ridges 22. In the embodiment illustrated, the ridges 22 are formed parallel to one another and are each oriented generally parallel to the plane defined by the plate member 12. The minor diameters of the ridges 22 define an effective opening having a circular shape.

The circular ridges 22 are configured to cooperate with a spherically shaped head of a bone screw. For example, the first bone screw 20A, which is shown particularly in Figures 2 and 3, is illustrated to include a spherically shaped threaded head 24 for engaging the ridges 22. Engagement of the threaded head 24 with the ridges 22 functions to orient the bone screw 20A relative to the plate member 12 and to fix the bone screw 20A relative to the plate member 12. Given the orientation of the circular ridges, the bone screw 20A is resultingly oriented with its longitudinal axis A₁ generally perpendicular to the plane defined by the plate member 12. In the embodiment illustrated, the head 24 of the first bone screw 20A includes a double lead thread 26. Alternatively, the ridges 22 of the plate member 12 can be replaced with a helical thread (not shown with respect to the first hole 18A) for threadably engaging the head 24.

In certain applications, it may be desired to orient the ridges 22 at an angle relative to the plane of the plate member 12. In such alternative applications, the bone screws 20A would correspondingly be fixed to the plate member 12 at an angle. For example, certain applications may require convergence of two or more bone screws 20 at fixed angles.

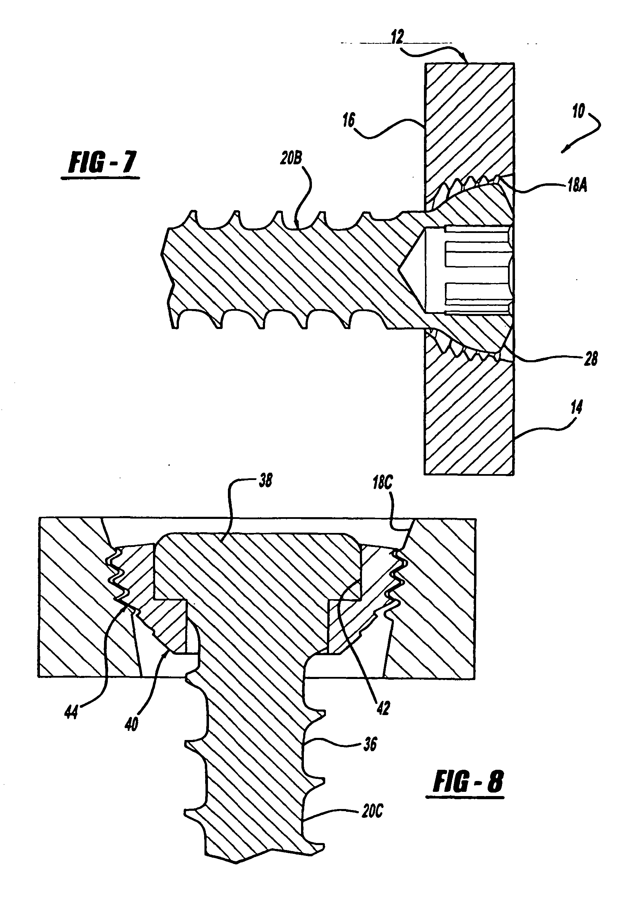

As shown in the cross-sectional view of Figure 7, the first hole 18A is also particularly adapted to receive a second bone screw 20B having a head 28 without threads. The head 28 of the second bone screw 20B is generally spherical in the shape. This spherical shape of the head 28 cooperates with the ridges 22 to allow the second bone screw 20B to angulate relative to the plate member 12. In this regard, as compared to the fixed relationship established with the threaded head 28 of the first bone screw 20A (as shown in Figures 2 and 3) the second bone screw 20B can be engaged with the bone at a variable angle.

The elongated shape of the first hole 18A provides a surgeon with increased flexibility for bone screw placement. In this regard, the surgeon may position the bone screw 20A or 20B anywhere along the length of the first hole 18A in a direction of the first dimension D₁. This flexibility in positioning of bone screws 20A or 20B relative to the plate member 12 is available regardless of whether the surgeon elects to use a bone screw 20A having a threaded head 24 for establishing a fixed relationship between the plate member 12 and the bone screw 20A at a predetermined angle, or a bone screw 20B having an unthreaded head 28 that allows angulation relative to the plate member 12.

The elongated shape of the first hole 18A additionally allows the surgeon to compress a fracture of the bone by translating the bone screw 20 along the hole 18A in a direction parallel to D₁. The circular ridges 22 permit such translation even where a threaded head is used, thereby retaining the locking relationship between the plate member 12 and the bone screw 20. Translation of the bone screw 20 is manually accomplished with an insertion tool (e.g., screw driver) that engages the head. Explaining further, the surgeon linearly advances the screw 20 along the hole 18A with the insertion tool. In certain alternative applications, it may be desirable to automatically compress the bone by angling the ends of the hole 18A. In such applications, a non-threaded head of a bone screw 20 would engage an end of the hole 18A and compress the bone as the non-threaded head transitions past the angled end of the hole 18A.

With particular reference to the cross-sectional view of Figure 4, a second hole defined by the plate member 12 is illustrated. As compared to the first hole 28, the second hole 20B is generally circular in shape. The hole 20B has an upper diameter D₃ at the upper surface 14 of the plate member 12 and a smaller diameter D₄ at the lower surface 16 of the plate member 12. Between the upper surface 14 and the lower surface 16 the second hole 20B includes a spherically shaped portion 30 having a plurality of threads 32. Alternatively, it will be understood that the plate member 12 can be formed with a plurality of circular ridges similar to the ridges 22 shown and described in connection with the first hole 18A.

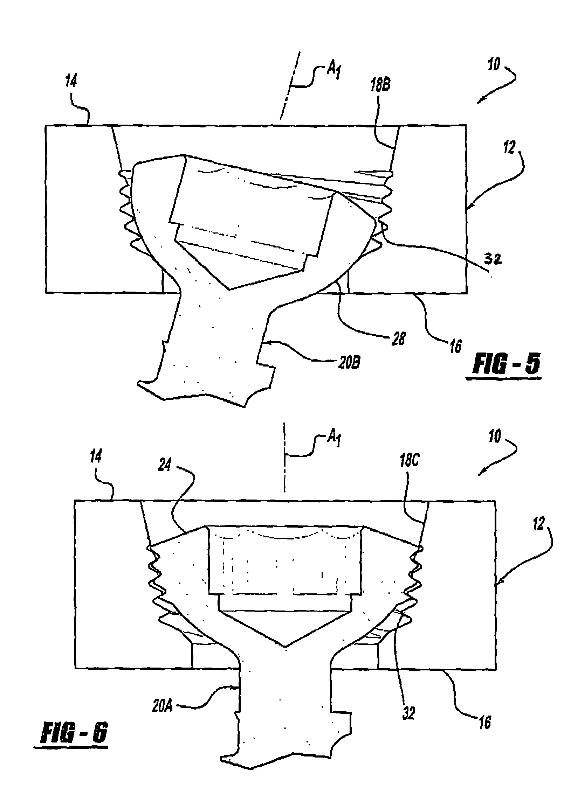

Figures 4 and 5 illustrate the second hole 20B operatively associated with a bone screw 20B identical to the bone screw 20B discussed above. Figure 4 illustrates the second bone screw 20B with its longitudinal axis oriented generally perpendicular to the plane defined by the plate member 12 immediately prior to seating of the spherical head 28 on the threads 32. Figure 5 illustrates the bone screw 20B seated with its spherical head 28 seated on the threads 32 and the bone screw 20B with its longitudinal axis articulated from the perpendicular orientation shown in Figure 4. It will be appreciated that the spherical shape of the head and the cooperating shape of the threads 32 allow the bone screw 20B to articulate from the orientation shown in Figure 4 approximately 15° in any direction.

With particular reference to the cross-sectional view of Figure 6, a third hole 20C defined by the plate member 12 is illustrated. It will be understood that the third hole 20C and the threads 32 associated with the third hole 20C are identical to the second hole 20B and corresponding threads 32 discussed above. The third hole 20C is illustrated with a bone screw 20A having a threaded head 24. The bone screw 20A is identical to the bone screw 20A discussed above. In the manner discussed above, the threaded head 24 and the threaded hole 20C cooperate for fixedly securing the bone screw 20A8 to the plate member 12. The bone screw 20A is secured to the plate with the longitudinal axis A₁ of the bone screw 20A oriented generally perpendicular to the plane defined by the plate member 12.

Turning to Figure 8, the third hole 18C of the plate member 12 is shown operatively associated with a third bone screw 20C. The third bone screw 20C is illustrated to include a threaded shaft portion 36 for threadably engaging a bone and a head portion 38. The third bone screw 20C is further shown to include a collar 40. The collar 40 defines an aperture 42 for receiving the head 38 of the third bone screw 20C. The collar 40 further includes an outer threaded surface 44 which is spherical in shape. The outer threaded surface 44 cooperates with the threads 32 of the hole 18C substantially in the manner discussed above with respects to Figure 6. The collar 40 maintains a locking connection with the plate member 12 while allowing the plate member 12 to be drawn adjacent the bone. In this manner, the profile of the plate member 12 may be minimized.

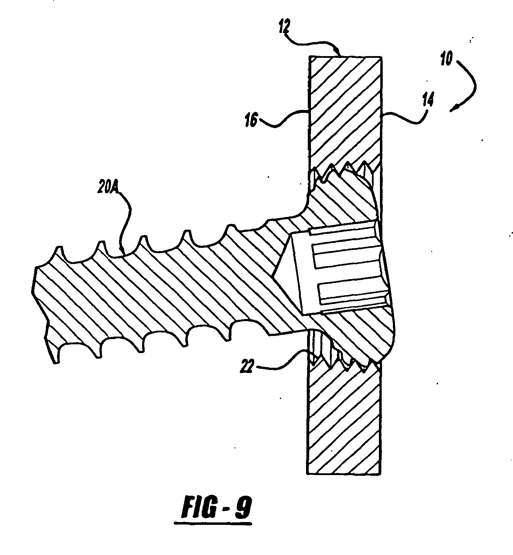

With particular reference to Figure 9, a cross-sectional view similar to Figure 3 is illustrated. In Figure 9, however, the bone screw 20A is angled from a perpendicular orientation. Such angulation of the bone screw 20A is permitted despite the threaded engagement between the head and the plate member 12 due to the double-lead thread of the head and the circular ridges 22 of the plate member 12.

In the embodiment illustrated, the double lead thread and the ridges 22 allow for angulation in increments of approximately 5° to 10° from the perpendicular orientation of Figure 3.

The teachings of the present invention have now been described to include various types of plate member holes and various types of bone screws. It is readily anticipated that the different holes and different bone screws can be combined alternatively for particular applications. Further in this regard, it is anticipated that certain applications may only include one type of hole. For example, a plate member may be constructed to include all elongated holes 18A. It is further anticipated that the various teachings of the present invention may be utilized separately in any combination to stabilize both long bones (including but not limited to the femur, the tibia, and the radius) and vertebral bodies.

The description of the invention is merely exemplary in nature and, thus, variations that do not depart from the scope of the appended claims are intended to be within the scope of the invention.

## Claims

1. A plating system (10) for bone, the plating system (10) comprising:
a plate member (12) having a top surface (14) and a bottom surface (16);
a plurality of holes (18) defined by the plate member (12) extending between the top surface (14) and the bottom surface (16) along a hole axis, at least a first hole (18A) of the plurality of holes having threads extending completely about an inner circumference, **characterised by** the first hole (18A) having a first dimension (D₁) generally perpendicular to the hole axis and a second dimension (D₂) generally perpendicular to the hole axis, the first dimension being greater than the second dimension, so that the first hole (18A) has an elongated shape; the plating system further comprising a bone screw (20A), whereby the elongated shape of the first hole (18A) enables positioning of the bone screw (20A) anywhere along the lengths of the first hole (18A) in a direction of the first dimension (D₁) and whereby the bone screw has an externally threaded spherical head threadably engaging the first hole (18A).

2. The plating system (10) for bone of Claim 1, wherein the first hole (18A) includes at least one ridge (22) extending completely about the inner circumference.

3. The plating system (10) for bone of Claim 2, wherein the first hole (18A) includes a plurality of circular ridges (22) extending completely about the inner circumference.

4. The plating system (10) for bone of Claim 2, wherein the head includes a double lead thread engaging the at least one ridge (22).

5. The plating system for bone of Claim 5, wherein the at least one ridge (22) is oriented generally perpendicular to the axis and the bone screw (20A) has a longitudinal axis angled relative to the hole axis.

6. The plating system (10) for bone of Claim 1, further comprising a second bone screw (20), which bone screw (20) has a shaft extending below the plate member (12) and a head disposed in a second hole (18C), the head being spherical, the head including a modular collar, the collar (40) having an outer spherical surface (44) threadably engaging the second hole (18C) of the plurality of holes (18), the second hole (18C) having a spherical and threaded inner surface.

7. The plating system of claim 6 wherein the second hole (18C) is helically threaded.

8. The plating system according to any of the preceding claims, wherein the plurality of holes are aligned to lie on an imaginary straight line extending longitudinally along the plate member.

## Patentansprüche

1. Ein Plattierungssystem (10) für Knochen, wobei das Plattierungssystem (10) aufweist:
ein Plattenelement (12), das eine obere Fläche (14) und eine untere Fläche (16) aufweist,
eine Mehrzahl von Löchern (18), die durch das Plattenelement (12) definiert sind, die sich entlang einer Lochachse zwischen der oberen Fläche (14) und der unteren Fläche (16) erstrecken, wobei mindestens ein erstes Loch (18A) der Mehrzahl von Löchern Gewinde aufweist, die sich vollständig um einen Innenumfang herum erstrecken, **dadurch gekennzeichnet, dass** das erste Loch (18A) eine erste Abmessung (D₁), die im Wesentlichen senkrecht zu der Hochachse ist, und eine zweite Abmessung (D₂) hat, die im Wesentlichen senkrecht zu der Lochachse ist, wobei die erste Abmessung größer als die zweite Abmessung ist, so dass das erste Loch (18A) eine langgestreckte Form hat, wobei das Plattierungssystem ferner aufweist: eine Knochenschraube (20A), wobei die langgestreckte Form des ersten Lochs (18A) ein Positionieren der Knochenschraube (20A) an irgendeiner Stelle entlang der Länge des ersten Lochs (18A) in einer Richtung der ersten Abmessung (D₁) ermöglicht, und wobei die Knochenschraube einen außen mit einem Gewinde versehenen sphärischen Kopf aufweiset, der mit dem ersten Loch (18A) in Gewindeeingriff ist.

2. Das Plattierungssystem (10) für Knochen gemäß Anspruch 1, wobei das erste Loch (18A) mindestens einen Grat (22) aufweiset, der sich vollständig um den Innenumfang herum erstreckt.

3. Das Plattierungssystem (10) für Knochen gemäß Anspruch 2, wobei das erste Loch (18A) eine Mehrzahl von kreisförmigen Geraten (22) aufweist, die sich vollständig um den Innenumfang herum erstrecken.

4. Das Plattierungssystem (10) für Knochen gemäß Anspruch 2, wobei der Kopf ein Doppelführungsgewinde aufweist, das mit dem mindestens einen Grat (22) in Eingriff ist.

5. Das Plattierungssystem für Knochen gemäß Anspruch 5, wobei der mindestens eine Grat (22) im Wesentlichen senkrecht zu der Achse ausgerichtet ist und die Knochenschraube (20A) eine Längsachse hat, die bezüglich der Lochachse abgewickelt ist.

6. Das Plattierungssystem (10) für Knochen gemäß Anspruch 1, ferner eine zweite Knochenschraube (20) aufweitend, wobei die Knochenschraube (20) einen Schaft, der sich unter dem Plattenelement (12) erstreckt, und einen Kopf aufweist, der in einem zweiten Loch (18C) angeordnet ist, wobei der Kopf sphärisch ist, wobei der Kopf einen modularen Kragen aufweist, wobei der Kragen (40) eine sphärische Außenfläche (44) aufweist, die mit dem zweiten Loch (18C) der Mehrzahl von Löchern (18) in Gewindeeingriff ist, wobei das zweite Loch (18C) eine sphärische und mit einem Gewinde versehene Innenfläche aufweist.

7. Das Plattierungssystem gemäß Anspruch 6, wobei das zweite Loch (18C) spiralförmig mit einem Gewinde versehen ist.

8. Das Plattierungssystem gemäß irgendeinem der vorhergehenden Ansprüche, wobei die Mehrzahl von Löchern ausgerichtet ist, um auf einer imaginären geraden Linie zu liegen, die sich entlang dem Plattenelement in Längsrichtung erstreckt.

## Revendications

1. Système de plaque d'ostéosynthèse (10), le système de plaque (10) comprenant :
un élément de plaque (12) ayant une surface supérieure (14) et une surface inférieure (16) ;
une pluralité de trous (18) définis par l'élément de plaque (12) s'étendant entre la surface supérieure (14) et la surface inférieure (16) le long d'un axe de trou, au moins un premier trou (18A) de la pluralité de trous ayant des filetages s'étendant complètement autour d'une circonférence interne, **caractérisé par** le premier trou (18A) qui a une première dimension (D₁) généralement perpendiculaire à l'axe de trou et une deuxième dimension (D₂), généralement perpendiculaire à l'axe de trou, la première dimension étant supérieure à la deuxième dimension, de sorte que le premier trou (18A) a une forme allongée ; le système de plaque comprenant en outre une vis à os (20A), moyennant quoi la forme allongée du premier trou (18A) permet de positionner la vis à os (20A) n'importe où sur les longueurs du premier trou (18A) dans une direction de la première dimension (D₁), et moyennant quoi la vis à os a une tête sphérique extérieurement filetée mettant en prise par filetage le premier trou (18A).

2. Système de plaque d'ostéosynthèse (10) selon la revendication 1, dans lequel le premier trou (18A) comprend au moins une crête (22) s'étendant complètement autour de la circonférence interne.

3. Système de plaque d'ostéosynthèse (10) selon la revendication 2, dans lequel le premier trou (18A) comprend une pluralité de crêtes circulaires (22) s'étendant complètement autour de la circonférence interne.

4. Système de plaque d'ostéosynthèse (10) selon la revendication 2, dans lequel la tête comprend un double pas mettant en prise la au moins une crête (22).

5. Système de plaque d'ostéosynthèse (10) selon la revendication 5, dans lequel la au moins une crête (22) est orientée de manière généralement perpendiculaire par rapport à l'axe, et la vis à os (20A) a un axe longitudinal coudé par rapport à l'axe de trou.

6. Système de plaque d'ostéosynthèse (10) selon la revendication 1, comprenant en outre une seconde vis à os (20), laquelle vis à os (20) a une tige s'étendant au-dessous de l'élément de plaque (12) et une tête disposée dans un second trou (18C), la tête étant sphérique, la tête comprenant un collier modulaire, le collier (40) ayant une surface sphérique externe (44) mettant en prise par filetage le second trou (18C) de la pluralité de trous (18), le second trou (18C) ayant une surface interne sphérique et filetée.

7. Système de plaque selon la revendication 6, dans lequel le second trou (18C) est fileté de manière hélicoïdale.

8. Système de plaque selon l'une quelconque des revendications précédentes, dans lequel la pluralité de trous est alignée sur une ligne droite imaginaire s'étendant longitudinalement le long de l'élément de plaque.
